# EUROPEAN PATENT APPLICATION

(11) **EP 3 692 973 A1**
(43) Date of publication of application: **12.08.2020**
(21) Application number: 18905902.5
(22) Date of filing: 14.12.2018
(51) Int. Cl.: A61H 3/00, A61F 5/01, A61F 2/66

(54) **POSTERIOR UPRIGHT OF ANKLE FOOT ORTHOSIS, AND ANKLE FOOT ORTHOSIS**

(71) Applicant: Sawamura Prosthetics and Orthotics Service Co., Ltd., Chuo-ku Kobe-shi Hyogo 650-0047 (JP); Therapy Co., Ltd., Chuo-ku Kobe-shi Hyogo 650-0047 (JP)
(72) Inventor: SANO, Taichi, Chuo-ku, Kobe-shi Hyogo 6500047 (JP); KONISHI, Katsuhiro, Chuo-ku, Kobe-shi Hyogo 6500047 (JP)
(74) Representative: Zimmermann, Tankred Klaus
(86) International application number: PCT/JP2018/046078
(87) International publication number: WO 2020/121513

(57) **Abstract**

To make it possible to adjust degree of bending deformation of a posterior upright of an ankle foot orthosis so that a wearer can move his or her foot well.

It is a posterior upright 4 of an ankle foot orthosis 1 provided between a heel posterior portion of a footrest portion 2 on which a foot portion is placed and a lower leg mounting portion 3 mounted on a lower leg portion below a knee. In the posterior upright 4, a plurality of FRP sheets 5a to 5c are laminated, and an upper end portion and a lower end portion of the FRP sheets are joined by heat sealing. An upper end portion 4A is a portion connected to the lower leg mounting portion 3, and a lower end portion 4B is connected to the footrest portion 2. An intermediate portion between the upper end portion 4A and the lower end portion 4B has gaps S 1 and S2 between the FRP sheets 5a to 5c.

## Description

### Technical Field

The present invention relates to a posterior upright of an ankle foot orthosis and an ankle foot orthosis.

### Background Art

A patient who has a disability, in which the patient cannot freely move an ankle joint with his or her own intention, due to hemiplegia or peripheral nerve palsy caused by a stroke, cerebral hemorrhage, or cerebral infarction cannot move body weight smoothly since a toe drops during walking (foot drop), and has difficulty walking.

A patient with such a symptom of foot drop has trouble walking due to a toe being caught by the floor and the like. Therefore, an ankle foot orthosis has been used as an auxiliary tool so that walking close to normal walking is achieved.

In such an ankle foot orthosis, when an ankle joint is firmly fixed and both a plantar flexion motion and a dorsal flexion motion are blocked, the ankle joint becomes almost unmovable, causing walking to become unnatural.

It has already been known that an ankle foot orthosis that matches with a body shape of each disabled person can be provided promptly by configuring the ankle foot orthosis with three components, which are a foot placing body on which a foot portion can be placed, a lower leg cuff mounted on a lower leg portion of a disabled person, and a posterior upright connecting the lower leg cuff and the foot placing body, and by appropriately selecting these components so that ankle foot orthoses of different specifications are manufactured promptly (see, for example, Patent Literature 1).

According to Patent Literature 1, a hinge mechanism is provided between the posterior upright and the foot placing body, and between the lower leg cuff and the posterior upright, so that a plantar flexion motion (bending in a direction in which a toe drops) or a dorsal flexion motion (bending in a direction in which a toe is raised) of an ankle joint can be performed, and also a shift between the lower leg cuff and the calf during walking and the like can be absorbed.

### Citation List

### Patent Literature

Patent Literature 1: JP 2004-344297 A

### Summary of Invention

### Technical Problem

However, the technique described in Patent Literature 1, which uses two hinge mechanisms, has a complicated structure.

The inventor has made the present invention by arriving at an idea that, by improving a structure of a posterior upright, degree of bending deformation of the posterior upright itself can be adjusted without providing such a hinge mechanism, and a foot can be moved well.

An object of the present invention is to make the degree of bending deformation of a posterior upright of an ankle foot orthosis adjustable, so that a wearer can move his or her foot well.

### Solution to Problem

The invention according to claim 1 is directed to a posterior upright of an ankle foot orthosis that is provided between a footrest portion on which a foot portion is placed and a lower leg mounting portion mounted on a lower leg portion below a knee, in which a plurality of FRP sheets are laminated, and an upper end portion and a lower end portion of the FRP sheets are joined by heat sealing, the upper end portion is a portion connected to the lower leg mounting portion, and the lower end portion is a portion connected to a heel posterior portion of the footrest portion, and an intermediate portion between the upper end portion and the lower end portion has a structure having a gap between the FRP sheets.

In this manner, when the posterior upright is bent until the gaps between the FRP sheets disappear, the FRP sheets come into contact with each other and the rigidity is increased, and the posterior upright does not bend any further. Therefore, the posterior upright bends to some extent with respect to tilting of a shin part of a wearer in a front-rear direction, and can follow a relative displacement between a foot portion and a shin portion to some extent during walking of the wearer. Further, since the rigidity is secured in a twisting direction, the footrest portion does not shift to the left or right during walking. Therefore, the wearer can move his or her foot well.

Furthermore, by inserting a rigid sheet into the gap between the FRP sheets, it is possible to adjust the degree of bending deformation of the posterior upright itself according to the wearer.

As described in claim 2, the lower end portion is preferably provided with a screw hole so as to be screwed to a heel posterior portion of the footrest portion.

In this way, the lower end portion can be screwed to the heel posterior portion of the footrest portion with a screw through the screw hole, which simplifies manufacture.

As described in claim 3, the upper end portion is preferably provided with a screw hole so as to be screwed to the lower leg mounting portion.

In this way, the upper end portion can be screwed to the lower leg mounting portion with a screw through the screw hole, which simplifies manufacture.

As described in claim 4, the screw holes so as to be screwed are arranged vertically, the screw holes being an upper hole and a lower hole through which a screw is inserted when the screw is attached, and the upper hole is preferably an arc-shaped long hole around the lower hole.

In this way, it is possible to attach the footrest portion and the lower leg mounting portion to the posterior upright by adjusting the orientation of the footrest portion and the lower leg mounting portion.

As described in claim 5, each of the FRP sheets is preferably a CFRP sheet.

In this way, necessary strength is ensured.

As described in claim 6, the lower end portion preferably has a flat plate shape or a bifurcated shape whose lower end is divided.

In this way, the lower end portion is firmly connected to the heel posterior portion of the footrest portion.

The invention according to claim 7 is directed to an ankle foot orthosis including a footrest portion on which a foot portion is placed, a lower leg mounting portion mounted on a lower leg portion below a knee, and a posterior upright that is provided between the lower leg mounting portion and the footrest portion and extends upward from a heel posterior portion to be connected to the lower leg mounting portion, in which the posterior upright is the posterior upright according to any one of claims 1 to 6.

In this way, the posterior upright bends to some extent with respect to tilting of a shin part of a wearer in a front-rear direction, and can follow a relative displacement between a foot portion and a shin portion during walking of the wearer. Further, since the rigidity is secured in a twisting direction, the footrest portion does not shift to the left or right during walking. Therefore, the wearer can move his or her foot well. Furthermore, by inserting a rigid sheet into the gap between the FRP sheets, it is possible to adjust the degree of bending deformation of the posterior upright itself according to the wearer.

As described in claim 8, a wedge-shaped block is configured to be sandwiched between a screwing portion of the posterior upright and the footrest portion or the lower leg mounting portion.

In this way, attaching angles of the footrest portion and the lower leg mounting portion can be adjusted by sandwiching the wedge-shaped block. Advantageous Effects of Invention

According to the present invention, when the posterior upright is bent until the gaps between the FRP sheets disappear, the FRP sheets come in contact with each other to increase the rigidity, and the posterior upright does not bend any further. Accordingly, the posterior upright bends to some extent with respect to tilting of a shin part of a wearer in a front-rear direction, and can follow a relative displacement between the foot portion and the shin portion to some extent during waling of the wearer. Further, since the rigidity is secured in a twisting direction, the footrest portion does not shift to the left or right during walking. Therefore, the wearer can move his or her foot well.

Furthermore, by inserting a rigid sheet into the gap between the FRP sheets, it is possible to adjust the degree of bending deformation of the posterior upright itself according to the wearer.

### Brief Description of Drawings

FIG. 1 is a front view showing an ankle foot orthosis using a posterior upright according to an embodiment of the present invention.
FIG. 2 is a front view of the posterior upright.
FIG. 3 is a side view of the posterior upright.
FIG. 4 is a front view showing an ankle foot orthosis using a posterior upright, which is another embodiment.
FIG. 5 is a side view of the posterior upright.
FIG. 6 is a front view of the posterior upright.
FIG. 7 is a view similar to FIG. 2 of a variation of the posterior upright.
FIG. 8 is a front view showing another ankle foot orthosis using a posterior upright which is an embodiment of the present invention.

### Description of Embodiment

Hereinafter, an embodiment of the present invention will be described with reference to the drawings.

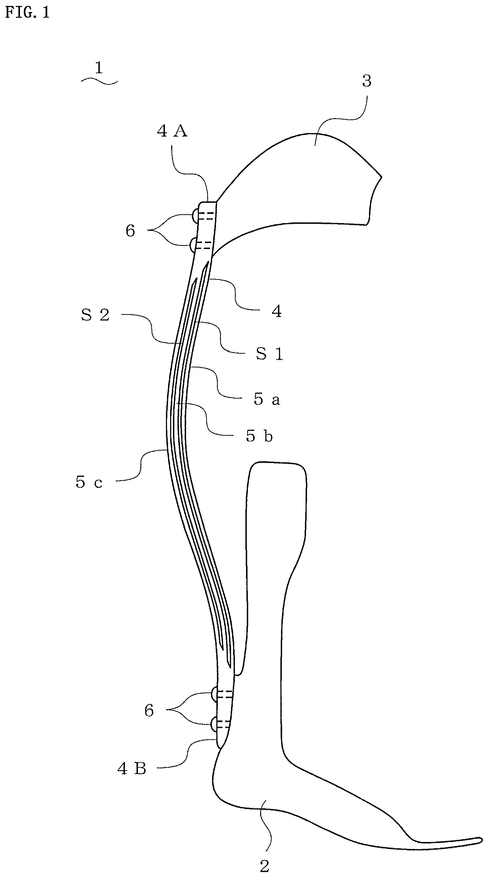

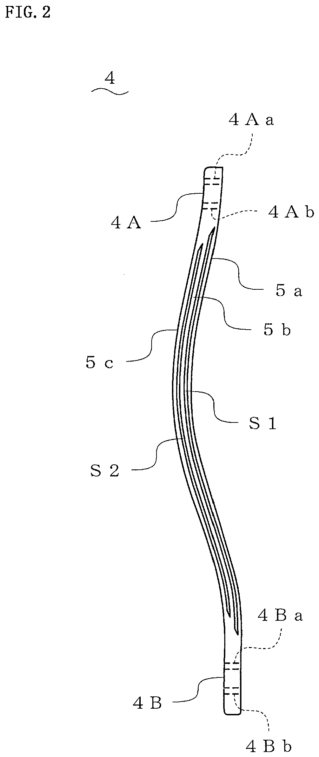

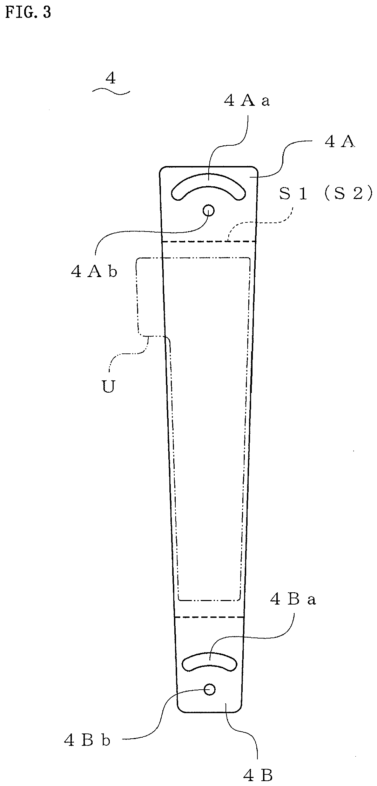

FIG. 1 is a front view showing an ankle foot orthosis using a posterior upright according to an embodiment of the present invention, FIG. 2 is a front view of the posterior upright, and FIG. 3 is a side view of the posterior upright.

As shown in FIG. 1, an ankle foot orthosis 1 includes a footrest portion 2 on which a wearer's foot portion is placed, a lower leg mounting portion 3 mounted on a lower leg portion below a knee of the wearer, and a posterior upright 4 that connects a heel posterior portion of the footrest portion 2 and the lower leg mounting portion 3. The posterior upright 4 extends upward from the heel posterior portion of the footrest portion 2, and is connected to a rear side portion of the lower leg mounting portion 3.

The posterior upright 4 has a laminated structure in which three FRP sheets 5a, 5b and 5c are laminated. Upper portions and lower portions of the FRP sheets 5a to 5c are joined by heat sealing to be integrated, and constitute an upper end portion 4A and a lower end portion 4B having a flat plate shape. Here, as the FRP sheets 5a to 5c, carbon fiber reinforced plastic sheets (CFRP sheets) are used.

Then, an intermediate portion between the upper end portion 4A and the lower end portion 4B is not heat-sealed, and has gaps S1 and S2 between the FRP sheets 5a to 5c in a state where no load is applied.

The upper end portion 4A is a portion connected to the lower leg mounting portion 3 and the lower end portion 4B is connected to the heel posterior portion of the footrest portion 2 by screwing.

The upper end portion 4A and the lower end portion 4B are provided with upper holes 4Aa and 4Ba and lower holes 4Ab and 4Bb as screw holes so as to be screwed to the lower leg mounting portion 3 and the heel posterior portion of the footrest portion 2 with screws 6. That is, the screw holes are arranged vertically, and the lower leg mounting portion 3 and the footrest portion 2 are attached by the screws 6 that are inserted through the upper holes 4Aa and 4Ba and the lower holes 4Ab and 4Bb when the screw is attached.

The upper holes 4Aa and 4Ba are arc-shaped long holes around the lower holes 4Ab and 4Bb, which allow the lower leg mounting portion 3 and the footrest portion 2 to be attached to the posterior upright 4 by adjusting the orientation of the lower leg mounting portion 3 and the footrest portion 2. The orientation can be adjusted within a range in which the long holes 4Aa and 4Ba extend.

If the ankle foot orthosis 1 having the posterior upright 4 as described above is mounted, when the posterior upright 4 bends until the gaps S1 and S2 between the FRP sheets 5a to 5c disappear, the FRP sheets come in contact with each other to become integrated and rigidity is increased, and the posterior upright 4 does not bend any further. As a result, the posterior upright 4 bends to some extent with respect to tilting of a shin part of the wearer in a front-rear direction, and can follow a relative displacement between a foot portion and a shin portion during walking of the wearer. In this manner, the wearer can perform a plantar flexion motion and a dorsal flexion motion of an ankle. Further, since rigidity is secured in a twisting direction, the footrest portion 2 does not shift to the left or right during walking. Therefore, the wearer can move his or her foot well.

Further, in a case where rigidity is desirably increased by reducing the degree of bending deformation, a rigid sheet U (see a two-dot chain line in FIG. 3) is inserted in the gaps S1 and S2 between the FRP sheets 5a to 5c in the intermediate portion between the upper end portion 4A and the lower end portion 4B. In this manner, the degree of bending deformation of the posterior upright 4 itself can be adjusted according to the wearer. That is, rigidity (the degree of bending deformation) of the posterior upright 4 can be changed in three stages by inserting the rigid sheet U into either or both of the gaps S1 and S2. Note that, if the number of FRP sheets is increased, it is possible to further increase the number of stages of rigidity adjustment.

In this manner, by inserting the rigid sheet U, it is possible to adjust rigidity of the posterior upright 4 according to the walking ability of the wearer, so that the wearer can move his or her foot well. Furthermore, the adjustment of the rigidity of the posterior upright 4, which can be performed only by inserting and pulling out the rigid sheet, is simple and can be performed in a short period of time.

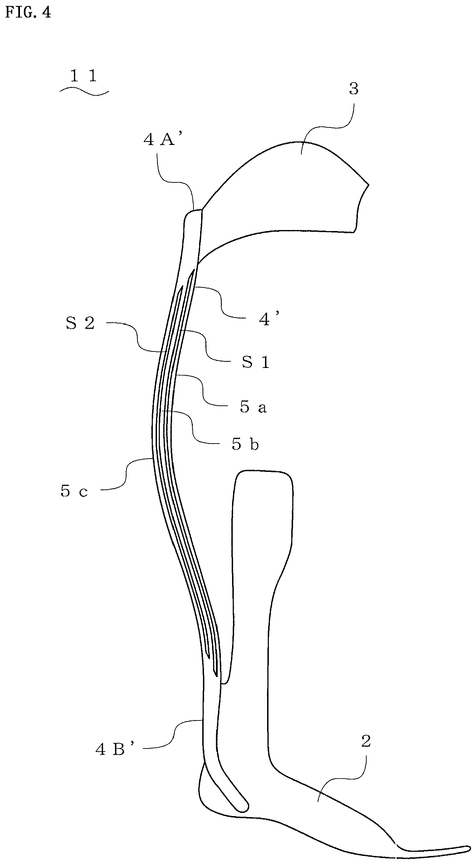

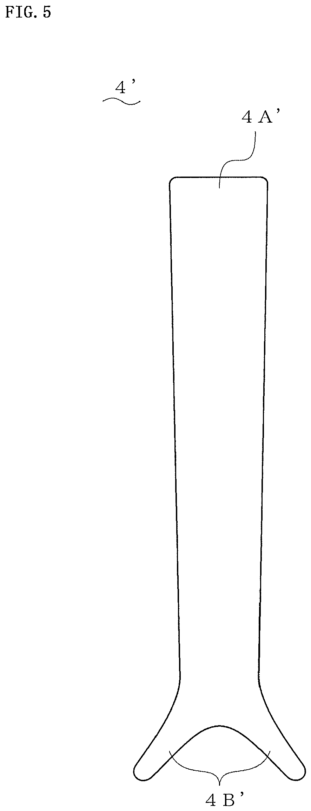

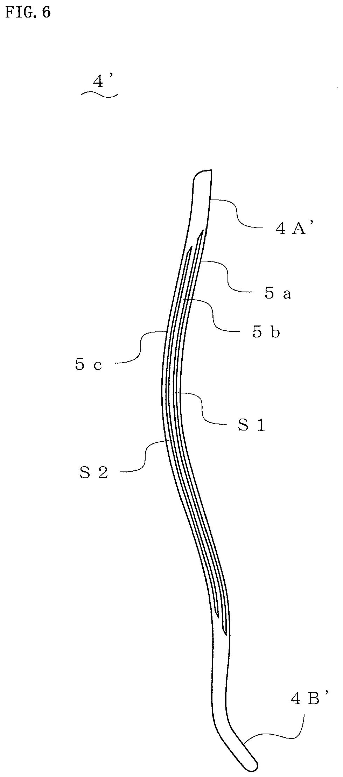

Although the upper end portion 4A and the lower end portion 4B of the posterior upright 4 are screwed to the lower leg mounting portion 3 and the footrest portion 2 in the above embodiment, the present invention is not limited to this configuration. For example, as shown in FIG. 4, in an ankle foot orthosis 11, an upper end portion 4A' and a lower end portion 4B' of a posterior upright 4' can also be connected to a rear side of the lower leg mounting portion 3 and the footrest portion 2 by heat sealing. In this case, the lower end portion 4B' of the posterior upright 4' is heat-sealed on both sides of a heel posterior portion of the footrest portion 2 as a bifurcated shape whose lower end is divided as shown in FIGS. 5 and 6.

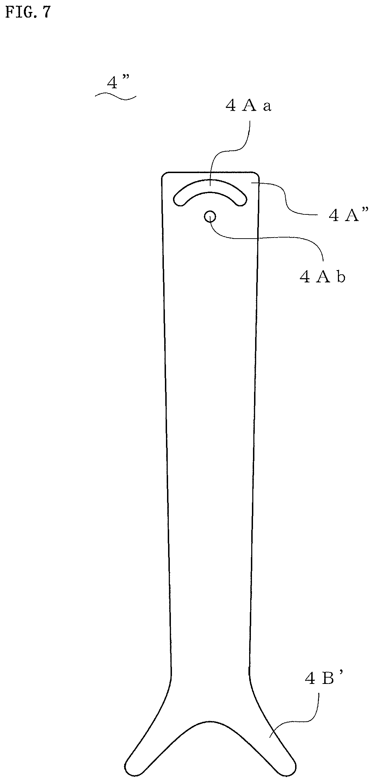

Further, as shown in FIG. 7, the upper hole 4Aa which is an arc-shaped long hole and the lower hole 4Ab which is a round hole can also be provided vertically in an upper end portion 4A" of a posterior upright 4", and the upper end portion can be screwed to the lower leg mounting portion 3.

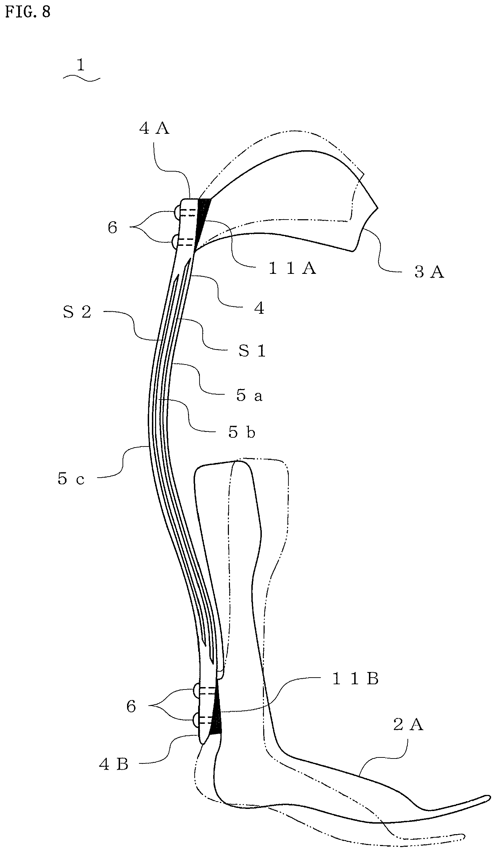

Furthermore, as shown in FIG. 8, wedge-shaped blocks 11A and 11B can also be sandwiched between screwing portions (the upper end portion 4A and the lower end portion 4B) of the posterior upright 4, and the footrest portion 2 and the lower leg mounting portion 3, so that attaching angles of a footrest portion 2A and a lower leg mounting portion 3A with respect to the posterior upright 4 can be adjusted. That is, if the blocks 11A and 11B are not used, a state shown by a two-dot chain line in FIG. 8 is obtained. However, by using the blocks 11A and 11B, attaching angles of the footrest portion 2A and the lower leg mounting portion 3A with respect to the posterior upright 4 can be adjusted and, by changing size and an inserting direction of the blocks 11A and 11B, the attaching angles can be finely adjusted.

### Reference Signs List

- 1: ankle foot orthosis
- 2: footrest portion
- 3: lower leg mounting portion
- 4, 4': posterior upright
- 4A, 4A': upper end portion
- 4Aa: upper hole
- 4Ab: lower hole
- 4B, 4B': lower end portion
- 4Ba: upper hole
- 4Bb: lower hole
- 5a, 5b, 5c: FRP sheet
- 6: screw
- 11A, 11B: block
- S1, S2: gap
- U: rigid sheet

## Claims

1. A posterior upright of an ankle foot orthosis that is provided between a footrest portion on which a foot portion is placed and a lower leg mounting portion mounted on a lower leg portion below a knee, wherein
a plurality of FRP sheets are laminated, and an upper end portion and a lower end portion of the FRP sheets are joined by heat sealing,
the upper end portion is a portion connected to the lower leg mounting portion, and the lower end portion is a portion connected to a heel posterior portion of the footrest portion, and
an intermediate portion between the upper end portion and the lower end portion has a structure having a gap between the FRP sheets.

2. The posterior upright of an ankle foot orthosis according to claim 1, wherein
the lower end portion is provided with a screw hole so as to be screwed to the heel posterior portion of the footrest portion.

3. The posterior upright of an ankle foot orthosis according to claim 1, wherein
the upper end portion is provided with a screw hole so as to be screwed to the lower leg mounting portion.

4. The posterior upright of an ankle foot orthosis according to claim 2 or 3, wherein
the screw holes so as to be screwed are arranged vertically, the screw holes being an upper hole and a lower hole through which a screw is inserted when the screw is attached, and
the upper hole is an arc-shaped long hole around the lower hole.

5. The posterior upright of an ankle foot orthosis according to any one of claims 1 to 4, wherein
each of the FRP sheets is a CFRP sheet.

6. The posterior upright of an ankle foot orthosis according to claim 1, wherein
the lower end portion has a flat plate shape or a bifurcated shape whose lower end is divided.

7. An ankle foot orthosis, comprising:
a footrest portion on which a foot portion is placed;
a lower leg mounting portion mounted on a lower leg portion below a knee; and
a posterior upright that is provided between the lower leg mounting portion and the footrest portion and extends upward from a heel posterior portion to be connected to the lower leg mounting portion, wherein
the posterior upright is the posterior upright according to any one of claims 1 to 6.

8. The ankle foot orthosis according to claim 7, wherein
a wedge-shaped block is sandwiched between a screwing portion of the posterior upright and the footrest portion or the lower leg mounting portion.
